# EUROPEAN PATENT SPECIFICATION

(11) **EP 0 626 015 B1**
(45) Date of publication and mention of the grant of the patent: **09.04.1997**
(21) Application number: 93904704.9
(22) Date of filing: 28.01.1993
(51) Int. Cl.: C12Q 1/26, C12Q 1/48, C12Q 1/54, C12Q 1/60, C12Q 1/62

(54) **INHIBITION OF CATALASE ACTIVITY IN BIOLOGICAL FLUIDS**
HEMMUNG DER KATALASEAKTIVITAET IN BIOLOGISCHEN FLUESSIGKEITEN
INHIBITION DE L'ACTIVITE D'UNE CATALASE DANS DES FLUIDES BIOLOGIQUES

(30) Priority: 31.01.1992 US 828453
(43) Date of publication of application: 30.11.1994
(73) Proprietor: ACTIMED LABORATORIES, INC., Mount Laurel, NJ 08054 (US)
(72) Inventor: WHITE, Mark, D., Sewell, NJ 08080 (US); LAW, Wai, Tak, Sewell, NJ 08080 (US)
(74) Representative: Plougmann, Vingtoft & Partners A/S
(86) International application number: PCT/US93/00771
(87) International publication number: WO 93/15218

(56) References cited:
- EP-A- 0 393 840
- US-A- 3 862 885
- US-A- 3 907 645
- US-A- 5 087 556
- DATABASE WPIL Section Ch, Week 8804, Derwent Publications Ltd., London, GB; Class B04, AN 88-021879
- DATABASE WPIL Section Ch, Week 8806, Derwent Publications Ltd., London, GB; Class A96, AN 88-039006
- PATENT ABSTRACTS OF JAPAN vol. 12, no. 264 (P-734)1988
- PATENT ABSTRACTS OF JAPAN vol. 2, no. 10 (E-6)1978

## Description

### FIELD OF THE INVENTION

The present invention is directed to an improvement in the assay art. It relates to methods and compositions for inhibiting catalase activity in biological fluids, and more specifically to inhibiting catalase activity in biological fluids during qualitative or quantitative analysis of biological fluids, when hydrogen peroxide participates in a signal-generating reaction.

### BACKGROUND OF THE INVENTION

Human serum contains many enzymes such as catalase, glutathione reductase and peroxidase, which readily decompose hydrogen peroxide to oxygen and water, the most important of these enzymes being serum catalase [EC 1.11.1.6]. The amount of catalase that is present in the plasma of normal human subjects can degrade hydrogen peroxide at a rate of 0.01 to 0.05 moles/liter/minute (Yamagata et al., Tohoku J. Exp. Med. 57:85-92, 1952; Yamagata et al., Tohoku J. Exp. Med. 57: 101- 107, 1953; Goth et al., Clin. Chem. 29: 741-743, 1983). Catalase is also present in erythrocytes in levels 3600 fold higher than in plasma, giving erythrocytes the capacity to degrade gram quantities of hydrogen peroxide over several minutes, Gaetani et al., Blood 73: 334-339 (1989). In fact, catalase has been used to verify the specificity of assays of hydrogen peroxide in plasma or blood, and the degradation of hydrogen peroxide or inhibition of the assay system by the sample should be checked, as catalase present in blood or plasma can reduce the value of measured hydrogen peroxide to zero, Nahum et al., Free Radical Biology & Medicine 6: 470-484, 1989.

The ability to measure a wide variety of physiologically active compounds, both naturally occurring and synthetic, has become of increasing importance, both as an adjunct to diagnosis and as therapy. While for the most part assays of physiological fluids and drugs have required clinical laboratory determinations, there is an increasing awareness of the importance of being able to conduct assays in a physician's office and/or in the home. To be able to perform an assay outside of a clinical laboratory setting requires that an assay have a simple protocol and be relatively free of sensitivity to small changes in the conditions under which the assay is conducted. Although a number of systems have been developed to try to address the problems associated with analysis outside of a clinical laboratory, there is nevertheless a continuing interest in providing improved and alternative methods to those which are presently generally available.

If a clinical detection reaction requires the presence of hydrogen peroxide at any stage of the assay, any endogenous serum catalase activity present in the reaction system will interfere with the reaction. Dilution of the sample to reduce catalase interference necessarily reduces the sensitivity of the reaction. Many clinical detection systems require the presence of hydrogen peroxide, including determinations of cholesterol, triglycerides, glucose, ethanol, lactic acid, etc. For example, when free cholesterol is oxidized by cholesterol oxidase to form cholestenone and hydrogen peroxide, the hydrogen peroxide so generated can be used to measure the amount of cholesterol originally in the sample.

One of the primary needs in clinical assays is the need to determine cholesterol or triglyceride, including high and low density lipoprotein, levels in blood. There is a clear relationship between total blood cholesterol (mainly the LDL fraction) and coronary artery disease (Journal of the American Medical Association 253: 2080-2086, 1985). New guidelines have been established for adults over 20 years of age to identify risk groups associated with blood cholesterol level, wherein less than 200 mg/dl is a desirable blood cholesterol; 239 mg/dl is borderline high blood cholesterol, and more than 240 mg/dl is considered to be high blood cholesterol and thus the patient is considered to be high risk.

Because cholesterol levels can be controlled by both diet and cholesterol lowering drugs for those patients at risk, the ability to monitor one's own cholesterol at home for those individuals at risk provides an important tool for monitoring cholesterol levels and thus reducing the potential for heart disease. The ability to measure other naturally occurring compounds of physiological importance, as well as levels of synthetic drugs or hormones is also of great interest.

Detection of components in liquids, such as cholesterol in blood, by test strips is well known. Vogel et al., in U.S. Patent No. 4,312,834, and Goodhue et al., in U.S. Patent No. 3,983,005, disclose test strips which can be used for detecting cholesterol in serum. Problems associated with catalase activity in human serum are avoided by including competing enzymes such as peroxidases in large excess so that the effects of catalase are minimized.

Allen et al., in U.S. Patent No. 4,999,287, disclose stripsticks for direct assay of physiologically active compounds such as cholesterol wherein the adverse effects of catalase are lessened by diluting the sample.

EP-A-0 393 840 (Allen) discloses adding sodium azide as a catalase inhibitor to biological assays to inhibit the action of catalase. No other inhibitors are suggested.

US-A-3,907,645 (Richmond) discloses adding an azide such as sodium azide to function as a catalase inhibitor in cholesterol assays wherein hydrogen peroxide is produced. No other catalase inhibitors are taught.

US-A-3,862,885 (Kano et al.) discloses uricase assays which generate hydrogen peroxide into which a catalase inhibitor is incorporated. The catalase inhibitors disclosed are azides and cyanides.

DD-A-249154 discloses test agents for assaying body fluids based upon an oxidase/peroxidase system and using aromatic diamines or benzidines as colour developers, and a colour coupler containing a mixture of hydroxylamine and at least one alkali or ammonium sulfate. The concentration of hydroxylamine is tied to the concentration of the colour developers, so that it appears that the hydroxylamine is included as part of the colour developing system rather than as a catalase inhibitor. There is no hint of using the hydroxylamines as other than colour developers.

JP-A-62-296897 discloses an analytic element containing oxidase and a hydrogen peroxide-detecting colour reagent. Catalase activity inhibitor is contained in a substantially inactivated form in a layer more close to the developing layer than the layer containing the hydrogen peroxide-detecting colouring reagent. Among the catalase inhibitors mentioned are azides, fluorine compounds, aminotriazoles, acetates, etc. There is no mention of hydroxylamines as catalase inhibitors.

JP-A-63-048454 discloses an analytic element incorporated into the reagent layer. The only examples of a catalase inhibitor given are a cyanic compound or an azide compound.

JP-A-52-124394 discloses a glucose determination in which a catalase inhibitor is added to the sample to prevent decomposition of hydrogen peroxide. The only catalase inhibitor disclosed is sodium azide.

In reaction systems where undiluted plasma samples are incorporated, the activity of endogenous enzymes may interfere with an analyte or precursor necessary to measure an analyte of interest. Additionally, many enzymes which are used as reagents in clinical assays may be contaminated with catalase or with other enzymes that destabilize hydrogen peroxide. In chemical reactions that utilize hydrogen peroxide as an oxidizing agent, or which utilize hydrogen peroxide to oxidize a dye or other intermediate to generate a colored or otherwise indicator species, the stability of hydrogen peroxide is an absolute necessity to provide an accurate measurement of the analyte of interest.

Enzymes have been incorporated into detergent compositions because of the enzymes' effectiveness against various common stains which are fixed to textiles and laundry. In particular, proteolytic enzymes which possess the ability to digest and degrade protein matter are used to remove from textiles proteinic stains such as blood, perspiration, milk, cocoa, gravy and other types of sauces. However, many of these detergent compositions also include peroxide and/or persulfate bleaching compounds, and catalase, which is present in many of these common stains, including blood, readily destroys these peroxide and persulfate bleaching compounds.

In order to minimize the effect of catalase on the bleaching compounds, inhibitor compounds can be incorporated in the detergent compositions, as disclosed in Gobert, U.S. Patent No. 3,751,222; Gobert et al., U.S. Patent No. 3,606,990, and Oukadi et al., U.S. Patent No. 4,753,750. The inhibitors may be contacted with the stained cloth during a soaking or prewashing stage prior to contact with the peroxide or persulfate bleaching agent or, alternatively, during the washing step, they may be included with the peroxide or persulfate bleaching agent. Of particular importance are compositions which comprise a mixture of inhibitor and detergent. These inhibitors are designed to be used in the presence of surfactants, including anionic, nonionic, amphoteric and cationic surfactants in the presence of peroxide and/or persulfate bleaching agents.

Among the inhibitors which have been found to be useful in detergent compositions for inhibiting the activity of catalase in soaking or washing solutions are hydroxylamine sulfate; hydroxylamine hydrochloride; phenylhydrazine; hydrazine; hydrazine sulfate; saturated phenol; polyphenols substituted witn at least one of NH₂, SO₂NH₂, Cl, Br, NO₂; aminophenols including o-amino-p-chlorophenol; aminotriazoles; alkali metal chlorates; sodium nitride; alkali metal cyanurates; and mixtures of the above.

Many of the above inhibitors used in detergent compositions are active only at extreme pH values, such as less than 4.0 or greater than 12.0, depending upon the type of detergent and/or bleach composition used. Additionally, many of these compounds are strong reducing agents, such as formaldehyde, which are not compatible with biological assay systems. Others of the inhibitors which are strong oxidizing agents, including alkali metal hypochlorites, alkali metal salts of chlorocyanuric acids, or potassium salts of monopersulfuric acid, may be strong denaturing agents. These properties may be acceptable in detergent formulations, but they are completely unacceptable in clinical assay systems in which the components of the systems are to be preserved, not destroyed. In addition, heavy metal salts such as mercuric chloride, nickel salts, or solvents such as acetone, either precipitate proteins or interfere with enzyme active site activity. Thus, many of these inhibitors cannot be used in diagnostic enzymatic reactions, in which the substances in the samples are not stable at extremes of pH or in the presence of many types of inhibitor compounds.

No admission is made that any of the background references cited above contributes prior art or pertinent prior art.

### SUMMARY OF THE INVENTION

It is an object of the present invention to overcome the aforesaid deficiencies in the background art.

It is a further object of the present invention to provide compositions for analysis of physiological fluids which are not inactivated in the reaction milieu by enzymes which decompose hydrogen peroxide.

It is another object of the present invention to provide compositions for analysis of physiological fluids which are not inactivated by catalase present in the physiological fluids.

It is still another object of the present invention to provide compositions for the analysis of physiological fluids which are not inactivated by catalase present in the reagents.

It is a further object of the present invention to minimize the adverse effects of catalase in an analysis system in which hydrogen peroxide is generated.

It is still another object of the present invention to minimize the adverse effects of catalase in reaction systems in which hydrogen peroxide is generated in serum in the absence of competitive enzymes such as peroxidases.

According to the present invention, inhibitors of enzymes which decompose hydrogen peroxide are used in clinical assays in which hydrogen peroxide is present in the sample or is subsequently generated the course of the assay. For example, the hydrogen peroxide may be used as an oxidizing agent, e.g., to oxidize a dye or other intermediate to generate a detectable species. These inhibitors may be provided in order to maintain the stability of the hydrogen peroxide.

Thus, for example, the addition of an effective amount of inhibitors of catalase or other such enzyme activity permits the use of undiluted plasma or serum in a diagnostic device without loss of the presence of hydrogen peroxide. Furthermore, the concentration of the inhibitor substance is sufficiently low and/or the activity of the inhibitor is sufficiently specific so that it does not substantially interfere with the assay; i.e., any interference attributable to the catalase inhibitor must be less than that derived from the catalase present in the sample.

The present invention is particularly advantageous in that undiluted serum or plasma samples may be used for the determination of analyte concentration. This is particularly important when the reaction format is such that hydrogen peroxide is generated in serum or plasma samples in the absence of competitive enzymes such as peroxidases. Most importantly, the inhibitors are effective inhibitors of catalase activity that do not interfere with enzymatic end point detections, including the Trinder type of enzymatic end point reactions for the quantitative measurement of cholesterol or triglycerides where the production of a visually detectable product species is proportional to the concentration of analyte in the sample.

In a preferred embodiment, hydroxylamine salts such as hydroxylamine sulfate and hydroxylamine hydrochloride are used to inhibit catalase activity. These compounds provide excellent inhibition of catalase activity in serum when the inhibitors are present in very low concentrations, e.g., in the range of about 0.5-40.0 mM in the reaction solution. Moreover, these inhibitors do not interfere with the several commonly used reporter enzymes which may be present in the system, including cholesterol esterase and cholesterol oxidase, and, for all practical purposes, horseradish peroxidase. Thus, the presence of the hydroxylamine salt does not interfere with the course or the result of the reaction. This reaction format is particularly advantageous because catalase interference is eliminated before the subsequent reaction steps occur, thus permitting an accurate determination of the analyte of interest.

### DETAILED DESCRIPTION OF THE INVENTION

This invention pertains generally to the assay use of inhibitors of enzymatic decomposition or conversion of hydrogen peroxide which do not substantially interfere with other enzymes in the assay system, including analytes, binding agents and labels.

In a preferred embodiment, the inhibitors are catalase inhibitors. The catalase inhibitors used in the present invention should more strongly inhibit the action of catalase but not of other important enzymes used in the clinical assays. Preferably, the other enzymes are not substantially inhibited, i.e., there is no measurable inhibition of these enzymes so that the assay is not adversely affected, and the inhibitors do not substantially interfere with the assay reactions to be conducted.

Applicants have developed an assay for catalase inhibition which permits the ready identification of catalase inhibitors. The screening assay is readily adapted to identification of inhibitors of other H₂O₂ inactivating enzymes. It is believed that the mechanism of catalase activity inhibition by the hydroxylamine compounds is that of competitive inhibition of the catalase activity. (However, applicants are not bound to this theory, and their invention is not limited to inhibitors which so function.)

Compounds having structures similar to hydrogen peroxide were initially screened for catalase inhibition in clinical assay systems using samples of aqueous hydrogen peroxide containing additional catalase along with a dye indicator/peroxidase system. The inhibitors were added along with the extra catalase, and spectrophotometric measurements at 514 nm were obtained.

The percent recovery of absorbance is defined as follows:$\text{% recovery of absorbance =} \frac{\text{Absorbance inhibitor solution}}{\text{Absorbance blank solution}} \text{x 100%}$

In the following tables, the concentrations of inhibitor substances are expressed as the final concentration in the reaction system after accounting for dilution factors.

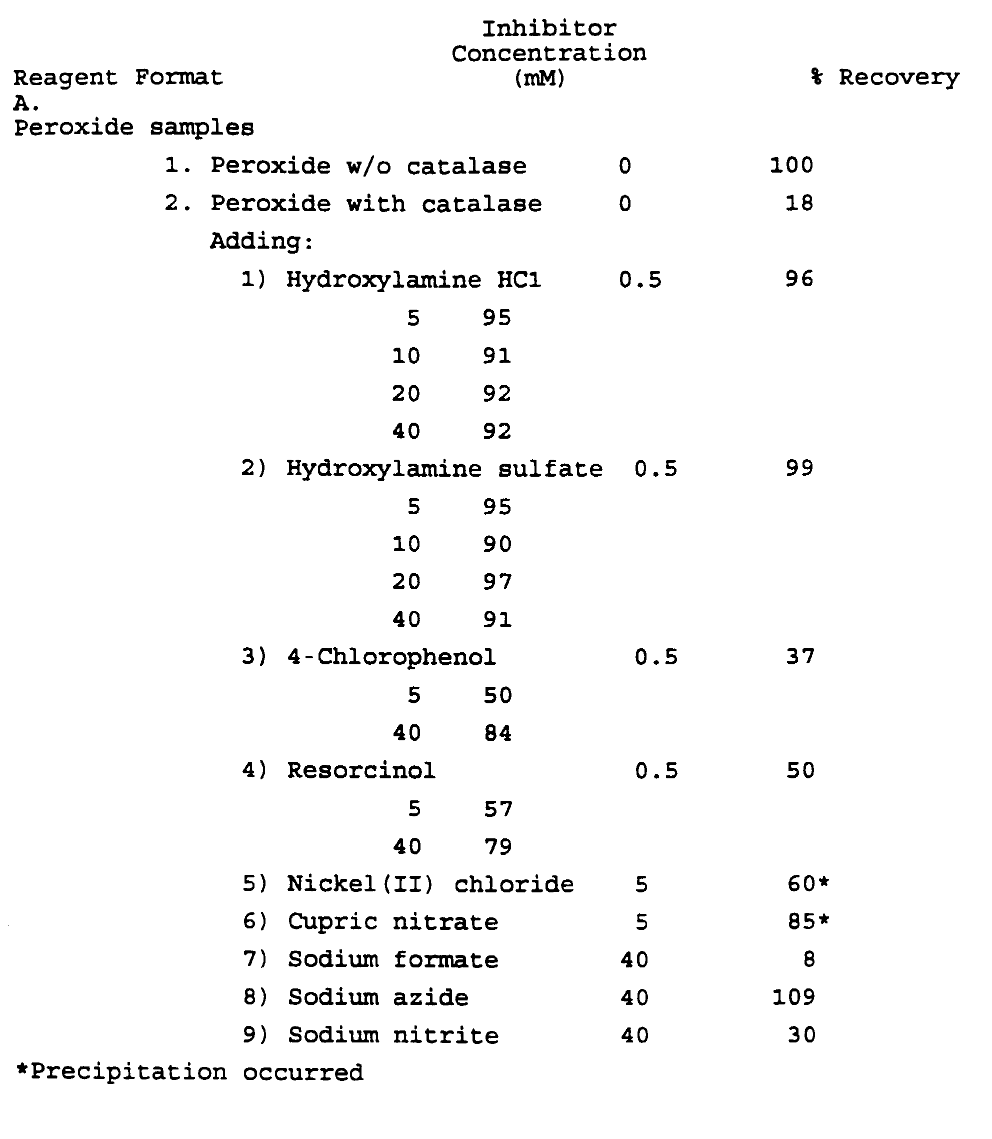

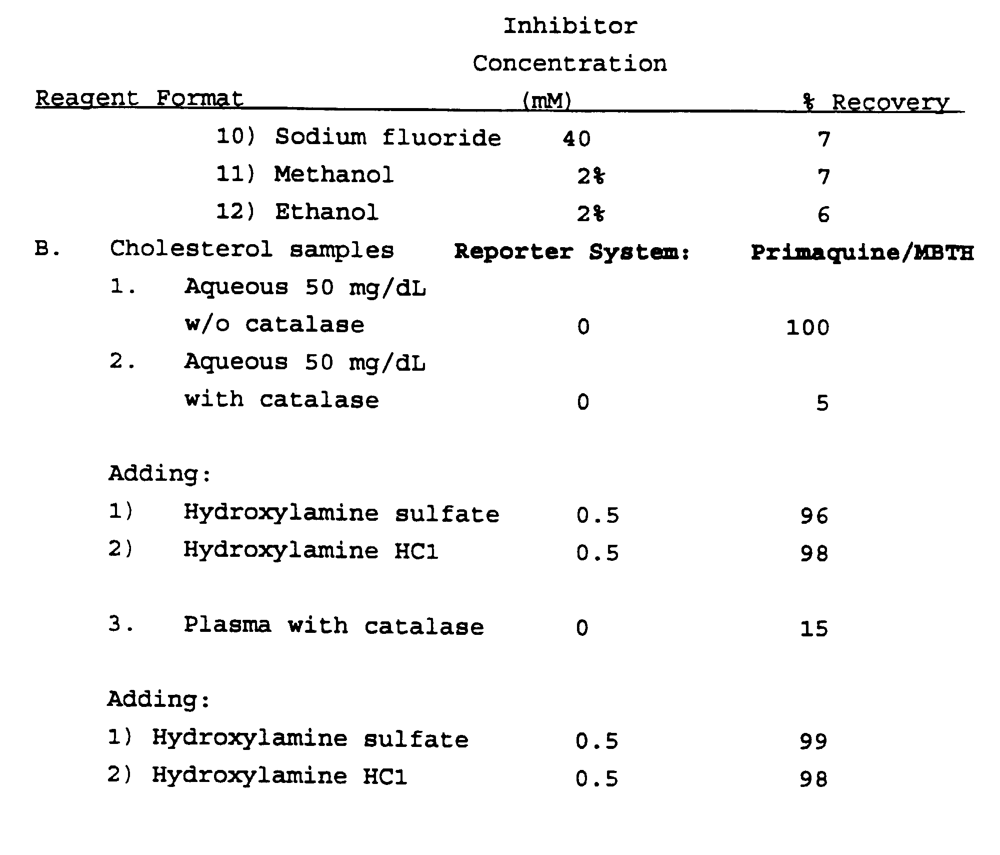

Preferably, for a particular assay system, use of a particular catalase-inhibitory amount of a catalase inhibitor achieves at least an 90% recovery in absorbance relative to the same system without the inhibitor. More preferably, it is at least 95%, and still more preferably, it is at least 97%. An effective amount of the catalase inhibitor is an amount that permits use of undiluted sample in a diagnostic device without loss of the presence of hydrogen peroxide. This effective amount may be determined by choosing a starting dosage, measuring the degree of inhibition of catalase and of other enzymes in the assay system, and then increasing or decreasing the dosage accordingly. The amount of inhibition achieved must be about 90% to be useful in reducing interference from catalase.

It is desirable that the inhibitor be substantially more specific for the catalase (or other contaminant enzyme) than for the competing enzymes (e.g., analytes, binding agents, or reporter enzymes). Preferably, the degree of inhibition of the contaminant enzyme is at least 50% greater than the degree of inhibition of the competing enzymes. More preferably, it is at least twice as great.

The above tables show the results of inhibitor screening experiments performed on potential inhibitors of catalase using a variety of reaction formats. Both of these tables illustrate that the hydroxylamine compounds are the most effective inhibitors of catalase activity. It should be noted that, in most instances, the recovery of absorbance with the hydroxylamine compounds is greater than 95%, and in a preferred embodiment, the inhibitor provides at least this degree of recovery. The screening assay may be extended by determining the effect of these inhibitors on other enzyme whose function is important to the ultimate assay.

The inhibitors of the present invention can be incorporated into any reagent system for use wherein hydrogen peroxide is generated in serum in the absence of competitive enzymes such as peroxidases. Of particular importance are assays for cholesterol or triglycerides, such as those disclosed in U.S. Patents 4,312,834 and 3,983,005. Additional assays which depend upon the generation of hydrogen peroxide include enzymatic assays for glucose, uric acid, lactic acid, free fatty acids, glutamate-pyruvate transaminase, glutamate- oxaloacetate transaminase, creatine phosphokinase, lactate dehydrogenase, cholinesterase, creatinine and the like.

The inhibitors of this invention are employed in an inhibitory amount, which may be determined by choosing a starting dosage, measuring the degree of inhibition of both catalase and of other enzymes in the assay system, and then increasing or decreasing the dosage accordingly. The amount of inhibition achieved needs to be above about 90% to be useful in reducing interference from catalase. The inhibitor may be added directly to the sample, or first to a reagent employed in the assay. The inhibitor may be added before the assay begins or after the addition of one or more reagents, but of course is preferably added before catalase has the opportunity to act on hydrogen peroxide present in the assay system.

Other enzymes which act on hydrogen peroxide and which may be desirable to suppress in an analytical environment include superoxide dismutase, glutathione peroxidase and glucose-6-phosphate dehydrogenase.

### Example 1

This example provides an example of quantitative determination of cholesterol in whole blood, using an assay device as disclosed in Ertingshausen, et al., U.S. Patent No. 5,087,556.

Assay devices according to Ertingshausen et al., U.S. Patent No. 5,087,556, are self-contained, chromatic quantitative analyzers that quantitatively detect and analyte in a fluid sample. The device includes a base ha ing a first open reservoir for receiving the fluid sample, with a means for separating solids from the biological fluid. A channel is provided which drawn, by capillary and/or wicking action, the biological fluid from the first open reservoir to a second open reservoir. The second open reservoir draws the biological fluid from the channel and, when the second open reservoir is filled with the fluid sample, the capillary and/or wicking action terminates. A membrane is provided in the channel which is permeable to the biological fluid. There is at least one chromatic chemical indicator immobilized in the membrane in a predetermined concentration. The membrane enables the biological fluid to interact with the chromatic chemical indicator.

The following enzyme reagent was prepared for application to a membrane channel:

| | General Range | Approximate | |
|---|---|---|---|
| | | Preferred Composition | amount per strip area (m²) |
| cholesterol esterase | 100-1500 U | 600 U | 6000U |
| cholesterol oxidase | 100-1500 U | 400 U | 4000U |
| sodium cholate | 1-50 mg | 10 mg | 100 mg |
| ascorbic acid oxidase | 10-500U | 100U | 1000U |
| Triton X 100 | 1-100 µL | 10 µL | 100 µL |
| Potassium hydrogen phosphate | 0.1-0.6 g | 0.3 g | 3 g |
| disodium hydrogen phosphate dihydrate | 0.1-0.6 g | 0.85 g | 8.5 g |
| deionized water | 10 ml | 10 ml | |
| hydroxylamine sulfate | 0.001-100 mg | 1 mg | 10 mg |

The following dye reagents were prepared for application to the channel:

| | General Range | Preferred Composition | Approximate amount per strip area (m²) |
|---|---|---|---|
| Primaquine phosphate - cellulose | 1 - 10 g | 4 g | 40 g |
| MBTH 3-Methyl-2 benzotiazolinone | 5 - 40 mg | 30 mg | 300 mg |
| hydrazone | | | |
| hydrochloride | | | |
| hydrate | | | |
| Polyvinyl alcohol | 1 - 3 g | 1.5 g | 15 g |

The following peroxidase reagents were applied to the channel:

| | General Range | Preferred Composition | Approximate amount per strip area (m²) |
|---|---|---|---|
| peroxidase | 1000-50,000U | 10,000U | 100,000U |
| potassium dihydrogen phosphate | 0.1 - 0.6 g | 0.3 g | 3 g |
| disodium hydrogen phosphate dihydrate | 0.1 - 1.5 g | 0.85 g | 8.5 g |
| polyvinyl pyrrolidone or Triton X 100 | | 0.05 ml | 0.5 ml |

An assay strip was prepared as disclosed in Ertingshausen, U.S. Patent No. 5,087,556, which assay strip included a first open reservoir, a channel in which the indicating reaction takes place, the channel including membrane in which the reagent is impregnated, and a second open reservoir.

To make an assay device which can be used in the present invention, a sheet of membrane is fed from a membrane roll stock, and a sheet of polyethylene laminate roll stock feeds a sheet of polyethylene laminate into a roller means. The membrane and the polyethylene laminate are sealed together by a first heat seal means to form a laminated membrane. The laminated membrane passes through at least one liquid dispensing means. The dispensing means selectively spray dye, enzymes, or other chromatic chemical indicators onto the membrane portion of the laminated membrane. The sprayed laminated membrane is then dried in a drying chamber.

A clear PVC roll stock feeds sheet PVC through a thermoform means, two hole punch means, and a draw zone and filter insert means. The filter-inserted PVC is laminated with the laminated membrane in a second heat seal means. The membrane is enclosed between the polyethylene laminate and the PVC upon exiting the second heat seal means to form an enclosed membrane. The enclosed membrane passes through a printer means where a scale is printed onto the enclosed membrane next to the channel.

A foil roll stock feeds a foil to the PVC side of the enclosed membrane. The foil provides a peelable, protective packaging for the device of this intention. Individual devices are cut from one another in a cutting stating. An electronic monitoring means is desirably provided to regulate, via appropriate continuous feedback control means, the operation of the various elements of the manufacturing equipment.

In conducting an assay for cholesterol using the device including the reagents as given above, whole blood was transferred into thefirst open reservoir of the device. The device also included, in the first open reservoir, means for separating solids from the plasma. The cell free or cell poor plasma entered the membrane channel in which plasma cholesterol was converted to cholestanone and hydrogen peroxide. An effective amount of hydroxylamine sulfate was present in this membrane channel to inhibit catalase destruction of the hydrogen.

The hydrogen peroxide entered the channel, which contained a precise amount of dye immobilized on a membrane. In the presence of horseradish peroxidase, the dye was quantitatively oxidized by hydrogen peroxide and converted to a colored species. The dye was evenly distributed in the membrane and its conversion occurred immediately upon contact with hydrogen peroxide. Therefore, the length of the color converted area was proportional to the amount of hydrogen peroxide formed and, therefore, to the amount of cholesterol in the sample.

Plasma devoid of hydrogen peroxide entered the pull chamber While the pull chamber was being filled, the oxidation of the dye in the channel continued until the pull chamber was completely filled, at which time the process stopped. The length of the color bar formed in the chamber was read from a scale which had been calibrated in cholesterol concentration units.

### Example 2

This example shows the effect of hydroxylamine salts on the length of color bar formed, which indicates the quantity of cholesterol in the sample. The results are shown in the following table.

| Plasma Cholesterol | Color Bar without HA | Length with HA |
|---|---|---|
| 200 mg/dL | 0 cm | 2.3 cm |
| 400 mg/dL | 0 cm | 4.9 cm |

### Example 3

The following example illustrates the reagents necessary to manufacture devices for quantitatively analyzing triglycerides in whole blood:

| | General Range | Preferred Composition | Approximate amount per strip area (m2) |
|---|---|---|---|
| glycerol kinase | 100-10,000U | 2000 U | 20,000U |
| lipase | 500-100,000 U | 10,000 U | 100,000U |
| glycerol phosphate oxidase | 100-50,000U | 12,000U | 120,000U |
| ATP | | 40 µmole | 0.4 µmole |
| Triton X100 (surfactant) | | 20 µL | 200 µL |
| Magnesium sulfate | | 20 µM | 0.2mM |
| NaCl | | 200 µM | 2 mM |
| Pipes buffer | | 50 µM | 0.5 mM |
| Sodium azide | | 1 mg | 10 mg |
| Deionized water | | 10 mL | |

The device prepared from the above formulation is used in the same manner as in Example 1.

### Example 4

| | General Range | Preferred Composition | Approximate amount per strip area (m2) |
|---|---|---|---|
| glucose oxidase | 100 - 30,000U | 6,000U | 60,000U |
| citric acid | 0.01 - 1 g | 0.3 g | 3 g |
| sodium citrate | 0.1 - 2 g | 1.0 g | 10 g |
| hyroxylamine hydrochloride | 0.1 - 10 mg | 1.0 mg | 10 mg |
| deionized water | | 10 mL | |

The device prepared from the above formulation is used in the same manner as Example 1.

The catalase inhibitors of the present invention can be used with any type of clinical assay in which hydrogen peroxide is produced or where hydrogen peroxide is present in the sample or in the reagent. The catalase inhibitor eliminates any uncertainty that may be present in the assay because of catalase destruction of hydrogen peroxide.

Although the catalase inhibitor of the present invention can be used in any type of assay in which hydrogen peroxide is either present or produced, integral analytic elements incorporating a reagent layer are particularly useful for diagnostics.

The inhibitors of the present invention can be used with any type of reagent which is currently used for clinical assays which produce hydrogen peroxide as a reaction product, or in which hydrogen peroxide is otherwise present. For example, in cholesterol assays, reagents which hydrolyze cholesterol esters contained in a liquid sample, which decompose cholesterol, including cholesterol liberated by such hydrolysis, and to provide detectable changes related to the total cholesterol content of the liquid all can be used along with the catalase inhibitors of the present invention, with no adverse effects on the reaction.

For cholesterol quantification in aqueous solutions containing cholesterol and/or cholesterol esters, such as blood serum, indicators can be used which quantify the level of hydrogen peroxide generated in the oxidation of cholesterol. Indicator compositions for the detection of enzymatically generated hydrogen peroxide are well known in the art, particularly as indicator compositions in the enzymatic detection of glucose and uric acid. U.S. Patents Nos. 3,092,465 and 2,981,606 describe indicator compositions which are useful in cholesterol analyses and which can be used with the hydroxylamine compounds which inhibit catalase activity.

Hydrogen peroxide indicator compositions generally comprise a substance having peroxidative activity, preferably peroxidase, and an indicator material which undergoes a visible change, such as a color change, in the presence of hydrogen peroxide and oxygen. Alternatively, the indicator material may be one or more substances which undergo no substantial color change upon oxidation in the presence of hydrogen peroxide and peroxidase, but which in their oxidized form react with a color-forming or color-changing substance to give visible quantitative evidence of a chemical reaction.

A peroxidase is an enzyme which will catalyze a reaction in which hydrogen peroxide oxidizes another substance. The peroxidases are generally conjugated proteins containing iron porphyrin. Peroxidase occurs in horseradish, potatoes, fig tree sap and turnips (plant peroxidase); in milk (lacto peroxidase); and in white blood corpuscles (verdo peroxidase); as well as in some microorganisms. Certain synthetic peroxidases, such as those disclosed by Theorell and Maehyl in Acta. Chem. Scand., 4: 422-434, 1950, are also satisfactory. Other substances which may be used are hemin, methemoglobin, oxyhemoglobin, hemoglobin, hemochromogen, alkaline hematin, hemin derivatives, and certain other compounds which demonstrate peroxidative or peroxidase-like activity, namely, the ability to catalyze the oxidation of another substance by means of hydrogen peroxide and other peroxides.

Other substances which are not enzymes but which possess peroxidase-like activity include iron sulfocyanate, iron tannate, ferrous ferrocyanide, chromic salts (such as potassium chromic sulfate) absorbed in silica gel, etc.

The most commonly used indicators are those which exhibit a color change, such as color-forming substrates of peroxidase and peroxidase-like substances which produce a color in the presence of hydrogen peroxide and peroxidase. These substances include monoamines such as aniline and its derivatives ortho-toluidine, etc; diamines, such as ortho- phenylenediamine, N,N'-dimethylparapenylenediamine; phenols such as phenol per se, thymol, ortho-, meta- and para-cresols, alpha- and beta-naphthol; polyphenols such as catechol, guiacol, orcinol, pyrogallol, p,p-dihydroxydiphenyl and phloroglucinol; aromatic acids such as salicylic, pyrocatecic and gallic acids; leuco dyes such as leucomalachite green and leucophenolphthalein; colored dyes such as 2,6- diochlorophenolindophenol; biological substances such as epinephrine, flavones, tyrosine, dihydroxyphenylalanine and tryptophan; other substances such as gum guaiac, guaiaconic acid, potassium, sodium, and other water soluble iodides; and bilirubin; as well as particular dyes such as 2,2'-azine-di-[3-ethylbenzothiazoline-(6)-sulfonic acid] and 3,3'-diaminobenzidine.

In an assay for cholesterol free cholesterol is hydrolyzed from chloesterol esters. Chlolesterol oxidase is then added, and the action of oxygen on free cholesterol in the presence of cholesterol oxidase produces hydrogen peroxide and cholest-4-en-one. Thus, in analyzing a solution containing free cholesterol, a reagent solution containing cholesterol oxidase is used, which solution contains a suitable hydroxylamine or sodium azide to reduce catalase interference. Optimum pH conditions are between about 4.5 and 9.5, and preferably between about 7.0 and 8.0.

An indicator composition is used to quantify the amount of hydrogen peroxide generated in the oxidation. More examples of such assays are given in U.S. Patent No. 3,983,005, to Goodhue et al. According to Goodhue et al., integral elements are provided for analysis of total cholesterol in aqueous liquids containing cholesterol and/or cholesterol esters. The element comprises a spreading layer in fluid contact with a reagent layer and contains
a. a cholesterol ester hydrolyzing composition comprising lipase having cholesterol esterase acitivity and protease; and
b. cholesterol oxidase.

The various materials are disposed with in the element so that cholesterol is released when the cholesterol esters in the liquid sample are saponified by the hydrolyzing composition an dfree cholesterol is decomposed in the presence of cholesterol oxidase, to produce in the element a detectable change that is related, preferably quantitatively to the total cholesterol content of the liquid sample. The cholesterol oxidase and the cholesterol ester hydrolyzing compositions are preferably incorporated into the element as follows:
a. both in the reagent layer;
b. both in the spreading layer; or
c. the cholesterol ester hydrolyzing composition in the spreading layer and some or all of the cholesterol oxidase in the reagent layer.

The reagent layer optionally contains an indicator composition which can react with at least one decomposition product of cholesterol to produce in the element a detectable change such as a color change, related to the total cholesterol concentration of a liquid sample applied to the element.

While it is preferable that the inhibitor be sufficiently specific for catalase so as not to interfere with other enzymes used in the assay system, it is possible to first react the inhibitor with the catalase, remove the inhibitor- catalase complex, and then inactivate, if need be, residual inhibitor, prior to introducing any vulnerable enzymes into the assay system.

The foregoing description of the specific embodiments will so fully reveal the general nature of the invention that others can, by applying current knowledge, readily modify and/or adapt for various applications such specific embodiments without departing from the generic concept, and therefore such adaptations and modifications are intended to be comprehended within the meaning and range of equivalents or the disclosed embodiments. It is to be understood that the phraseology or terminology herein is for the purpose of description and not of limitation.

## Claims

1. A method for analyzing biological fluid samples for the presence of an analyte other than an enzyme which decomposes hydrogen peroxide comprising using hydrogen peroxide as an oxidizing agent to oxidize an intermediate to generate a detectable species and measuring said detectable species, comprising adding to said sample an inhibitor for said enzyme selected from the group consisting of hydroxylamine sulfate and hydroxylamine hydrochloride.

2. The method according to claim 1 wherein said enzyme is catalase.

3. The method according to claim 1 or 2 wherein said method is for determination of an analyte selected from the group consisting of cholesterol, triglycerides, glucose, high density lipoproteins, low density lipoproteins, uric acid, lactic acid, free fatty acids, glutamate-pyruvate transaminase, glutamate-oxaloacetate transaminase, creatine phosphokinase, lactate dehydrogenase, cholinesterase, and creatinine.

4. The method according to any of claims 1-3 wherein said assay is a cholesterol determination.

5. The method according to any of claims 1-4 wherein cholesterol esterase or cholesterol oxidase is used as a reagent, and said inhibitor in said amount does not substantially inhibit cholesterol esterase or cholesterol oxidase.

6. The method according to any of claims 1-5 wherein said assay is a triglyceride determination.

7. The method according to any of claims 1-6 wherein said assay is a determination of high density lipoproteins.

8. The method according to any of claims 1-7 wherein said intermediate is a dye.

9. The method according to any of claims 1-7 wherein said detectable species exhibits a colour change.

10. An integral analytic device for clinical assay of fluid samples comprising:
a first open reservoir;
a channel, said channel having incorporated therein an indicator composition which gives visible evidence of the presence of hydrogen peroxide and an effective amount of a catalase inhibitor to minimize the adverse effects of catalase to hydrogen peroxide in a fluid sample;
said catalase inhibitor being selected from the group consisting of hydroxylamine hydrochloride and hydroxylamine sulfate;
and a second open reservoir.

11. The method of claim 1 wherein the detectable species is measured by means which comprise use of peroxidase as an analytical reagent.

## Patentansprüche

1. Verfahren zum Analysieren von Proben einer biologischen Flüssigkeit auf die Anwesenheit eines anderen Analyten als eines Enzyms, das Wasserstoffperoxid spaltet, umfassend das Verwenden von Wasserstoffperoxid als ein oxidierendes Agens zum Oxidieren eines Zwischenprodukts, um eine nachweisbare Spezies zu bilden, und Messen der nachweisbaren Spezies, umfassend das Versetzen der Probe mit einem Inhibitor für das Enzym, der aus Hydroxylaminsulfat und Hydroxylaminhydrochlorid ausgewählt wird.

2. Verfahren nach Anspruch 1, wobei das Enzym Catalase ist.

3. Verfahren nach Anspruch 1 oder 2, wobei das Verfahren der Bestimmung eines Analyten dient, ausgewählt aus Cholesterin, Triglyceriden, Glucose, HDLs (*High Density Lipoproteins*)*,* LDLs (Low *Density Lipoproteins),* Harnsäure, Milchsäure, freien Fettsäuren, Glutamat-Pyruvat-Transaminase, Glutamat-Oxalacetat-Transaminase, Kreatin-Phosphokinase, Laktat-Dehydrogenase, Cholin-Esterase und Kreatinin.

4. Verfahren nach einem der Ansprüche 1 - 3, wobei es sich bei dem Assay um eine Cholesterin-Bestimmung handelt.

5. Verfahren nach einem der Ansprüche 1 - 4, wobei die Cholesterin-Esterase oder Cholesterin-Oxidase als ein Reagens verwendet wird und die Menge an Inhibitor im wesentlichen nicht die Cholesterin-Esterase oder die Cholesterin-Oxidase inhibiert.

6. Verfahren nach einem der Ansprüche 1 - 5, wobei es sich bei dem Assay um eine Triglycerid-Bestimmung handelt.

7. Verfahren nach einem der Ansprüche 1 - 6, wobei es sich bei dem Assay um eine Bestimmung von HDLs handelt.

8. Verfahren nach einem der Ansprüche 1 - 7, wobei das Zwischenprodukt ein Farbstoff ist.

9. Verfahren nach einem der Ansprüche 1 - 7, wobei die nachweisbare Spezies einen Farbwechsel zeigt.

10. Integrale analytische Vorrichtung für den klinischen Assay von flüssigen Proben umfassend:
einen ersten offenen Flüssigkeitsbehälter;
einen Zugangskanal, wobei der Zugangskanal eine Indikatorzusammensetzung, die einen sichtbaren Nachweis der Anwesenheit von Wasserstoffperoxid gibt, und eine wirksame Menge eines Catalase-Inhibitors, um die nachteiligen Wirkungen von Catalase auf Wasserstoffperoxid in einer flüssigen Probe zu minimieren, einschließt;
wobei der Catalase-Inhibitor ausgewählt ist aus Hydroxylaminhydrochlorid und Hydroxylaminsulfat;
und einen zweiten offenen Flüssigkeitsbehälter.

11. Verfahren nach Anspruch 1, wobei die nachweisbare Spezies durch Mittel gemessen wird, die die Verwendung von Peroxidase als ein analytisches Reagens umfassen.

## Revendications

1. Procédé pour analyser des échantillons fluides biologiques quant à la présence d'un analyte autre qu'une enzyme qui décompose le peroxyde d'hydrogène consistant à utiliser du peroxyde d'hydrogène en tant qu'agent d'oxydation pour oxyder un intermédiaire afin d'engendrer une espèce détectable et à mesurer ladite espèce détectable, comprenant l'addition d'un inhibiteur, choisi dans le groupe formé par le sulfate d'hydroxylamine et le chlorhydrate d'hydroxylamine, audit échantillon pour inhiber ladite enzyme.

2. Procédé selon la revendication 1, dans lequel ladite enzyme est la catalase.

3. Procédé selon la revendication 1 ou 2, dans lequel ledit procédé est destiné à la détermination d'un analyte choisi dans le groupe formé par le cholestérol, les triglycérides, le glucose, les lipoprotéines haute densité, les lipoprotéines basse densité, l'acide urique, l'acide lactique, les acides gras libres, la glutamate-pyruvate transaminase, la glutamate-oxaloacétate transaminase, la créatine phosphokinase, la lactate déshydrogénase, la cholinestérase et la créatinine.

4. Procédé selon l'une quelconque des revendications 1 à 3, dans lequel ledit dosage est une détermination du cholestérol.

5. Procédé selon l'une quelconque des revendications 1 à 4, dans lequel la cholestérol estérase ou la cholestérol oxydase sont utilisées en tant que réactif, et ledit inhibiteur dans ladite quantité n'inhibe pas sensiblement la cholestérol estérase ou la cholestérol oxydase.

6. Procédé selon l'une quelconque des revendications 1 à 5, dans lequel ledit dosage est une détermination des triglycérides.

7. Procédé selon l'une quelconque des revendications 1 à 6, dans lequel ledit dosage est une détermination de lipoprotéines haute densité.

8. Procédé selon l'une quelconque des revendications 1 à 7, dans lequel ledit intermédiaire est un colorant.

9. Procédé selon l'une quelconque des revendications 1 à 7, dans lequel ladite espèce détectable présente un changement de couleur.

10. Dispositif analytique intégral pour un dosage clinique d'échantillons fluides comprenant:
- un premier réservoir ouvert;
- un canal, ledit canal ayant incorporé dedans une composition indicatrice qui donne une évidence visible de la présence de peroxyde d'hydrogène et une quantité efficace d'un inhibiteur de catalase pour minimiser les effets nuisibles de la catalase envers le peroxyde d'hydrogène dans un échantillon de fluide;
- ledit inhibiteur de catalase étant choisi dans le groupe formé par le chlorhydrate d'hydroxylamine et le sulfate d'hydroxylamine;
- et un deuxième réservoir ouvert.

11. Procédé selon la revendication 1, dans lequel l'espèce détectable est mesurée par un moyen qui consiste à utiliser une peroxydase en tant que réactif analytique.
